# EUROPÄISCHE PATENTSCHRIFT

(11) **EP 2 413 710 B1**
(45) Veröffentlichungstag und Bekanntmachung des Hinweises auf die Patenterteilung: **04.01.2017**
(21) Anmeldenummer: 10710346.7
(22) Anmeldetag: 26.03.2010
(51) Int. Cl.: A23D 7/005, A23K 20/179, A23L 33/10, A23K 20/105, A23L 2/52, A61K 31/07

(54) **GEBRAUCHSFERTIGE, STABILE SUSPENSION TEILAMORPHER BETA-CAROTINPARTIKEL**
READY-TO-USE, STABLE SUSPENSION OF PARTIALLY AMORPHOUS BETA-CAROTIN PARTICLES
SUSPENSION STABLE, PRÊTE À L'EMPLOI, DE PARTICULES DE BÊTA-CAROTÈNE PARTIELLEMENT AMORPHES

(30) Priorität: 30.03.2009 EP 09156651
(43) Veröffentlichungstag der Anmeldung: 08.02.2012
(73) Patentinhaber: BASF SE, 67056 Ludwigshafen am Rhein (DE)
(72) Erfinder: KÖPSEL, Christian, 69469 Weinheim (DE); SAMBALE, Clemens, 67459 Böhl-Iggelheim (DE); HASSE, Andreas, 67256 Weisenheim/Sand (DE)
(74) Vertreter: BASF IP Association
(86) Internationale Anmeldenummer: PCT/EP2010/053965
(87) Internationale Veröffentlichungsnummer: WO 2010/112406

(56) Entgegenhaltungen:
- EP-A2- 0 410 236
- WO-A1-91/06292
- WO-A1-94/19411
- WO-A1-2010/040683
- DE-A1- 19 651 681
- DE-U1-202006 010 606

## Beschreibung

Die vorliegende Erfindung betrifft eine gebrauchsfertige, stabile Suspension teilamorpher β-Carotinpartikel, ein Verfahren zu ihrer Herstellung sowie ihre Verwendung als Zusatz zu Tierfuttermitteln, Lebensmitteln und Nahrungsergänzungsmitteln sowie kosmetischen und pharmazeutischen Mitteln.

Die Stoffklasse der Carotinoide klassifiziert man in zwei Hauptgruppen, die Carotine und die Xanthophylle. Im Unterschied zu den Carotinen, bei denen es sich um reine Polyen-Kohlenwasserstoffe handelt, wie beispielsweise β-Carotin oder Lycopin, kommen in den Xanthophyllen noch Sauerstoff-Funktionen wie Hydroxy-, Epoxy- und/oder Carbonylgruppen vor. Typische Vertreter dieser Gruppe sind u. a. Astaxanthin, Canthaxanthin, Lutein und Zeaxanthin.

Diese sowohl synthetisch zugänglichen als auch aus natürlichen Quellen isolierbaren Polyene stellen für die Lebens- und Futtermittelindustrie sowie für den pharmazeutischen Bereich wichtige Farb- und Wirkstoffe dar und sind z. B. wegen ihrer Provitamin-A Aktivität von Interesse.

Sowohl Carotine als auch Xanthophylle sind in Wasser unlöslich, während in Fetten und Ölen eine ebenfalls nur geringe Löslichkeit gefunden wird. Diese begrenzte Löslichkeit sowie die hohe Oxidationsempfindlichkeit stehen einer direkten Anwendung der durch chemische Synthese erhaltenen, relativ grobkörnigen Produkte in der Einfärbung von Lebens- und Futtermitteln entgegen, da die Substanzen in grobkristalliner Form nicht lagerstabil sind und nur schlechte Färbungsergebnisse liefern. Diese für die praktische Verwendung der Carotinoide nachteiligen Effekte wirken sich insbesondere im wässrigen Medium aus, da sie darin gänzlich unlöslich sind.

Nur durch gezielt hergestellte Formulierungen, in denen die Carotinoide in fein verteilter Form und gegebenenfalls durch Schutzkolloide oxidationsgeschützt vorliegen, lassen sich bei der Einfärbung von Lebensmitteln verbesserte Farbausbeuten erzielen. Außerdem führen diese in Futtermitteln verwendeten Formulierungen zu einer höheren Bioverfügbarkeit der Carotinoide, das heißt der Carotine oder Xanthophylle, und damit indirekt zu besseren Färbungseffekten z. B. bei der Eidotter- oder Fischpigmentierung.

Zur Verbesserung der Farbausbeuten und zur Erhöhung der Resorbierbarkeit bzw. Bioverfügbarkeit sind verschiedene Verfahren beschrieben worden, die alle das Ziel haben, die Kristallitgröße der Wirkstoffe zu verkleinern und auf einen Teilchengrößenbereich von kleiner 10 µm zu bringen.

Zahlreiche Methoden, u. a. beschrieben in Chimia 21, 329 (1967), WO 91/06292 sowie in WO 94/19411, bedienen sich dabei der Vermahlung von Carotinoiden mittels einer Kolloidmühle und erzielen damit zum Teil Partikelgrößen von weniger als 2 µm.

In WO 2007/003543 wird beispielsweise ein Mahlverfahren beschrieben, bei dem β-Carotin als Suspension durch Mahlen in Gegenwart von Saccharose oder Glucose und modifizierter Stärke auf eine Partikelgröße von etwa 0,6 µm zerkleinert wird und die Carotinoid-haltige Suspension anschließend in ein Trockenpulver überführt wird.

Neben den Mahlverfahren existieren eine Reihe von kombinierten Emulgier-/Sprüh¬ trocknungsverfahren, wie sie z. B. in DE-A-12 11 911 oder in EP-A-0 410 236 beschrieben sind.

Gemäß der europäischen Patentschrift EP-B-0 065 193 erfolgt die Herstellung von feinverteilten, pulverförmigen Carotinoidpräparaten dadurch, dass man ein Carotinoid in einem flüchtigen, mit Wasser mischbaren organischen Lösungsmittel bei erhöhten Temperaturen, gegebenenfalls unter erhöhtem Druck löst, das Carotinoid durch Mischen mit einer wässrigen Lösung eines Schutzkolloids ausfällt und anschließend sprühtrocknet.

Ein analoges Verfahren zur Herstellung von feinverteilten, pulverförmigen Carotinoidpräparaten wird in EP-A-0 937 412 unter Verwendung von mit Wasser nicht mischbaren Lösungsmitteln beschrieben.

In der Getränkeindustrie werden Zusatzstoffe in der Regel in Form von flüssigen Konzentraten den Getränken zugesetzt. Es werden sowohl flüssige Formulierungen von Farbstoffen, wie Carotinoiden, angeboten als auch entsprechende wasserlösliche, pulverförmige Formulierungen, aus denen im Produktionsprozess bei den Getränkeherstellern zunächst eine wässrige Dispersionen des Farbstoffes hergestellt wird.

In EP 0 239 086 werden Emulsionen eines in Öl gelösten Carotinoids beschrieben, wobei zur Stabilisierung der Öltröpfchen eine Mischung aus einem Ester einer langkettigen Fettsäure mit Ascorbinsäure und einem kaltwasserlöslichen Stärkeprodukt, wie beispielsweise Stärkeoctenylsuccinat, eingesetzt wird. Die Carotinoidkonzentration liegt in diesen Emulsionen zwischen 0,1 und 2 %.

In EP 0 551 638 werden stabile flüssige Emulsionen von fettlöslichen Vitaminen oder Carotinoiden hergestellt, wobei die äußere Phase Glycerin oder ein Glycerin-WasserGemisch ist und als Emulgator und Stabilisator ein Ester der Ascorbinsäure mit langkettigen Fettsäuren eingesetzt wird. Im Falle von β-Carotin zeichnen sich die Präparate durch einen strahlenden Gelbton aus.

Die WO 93/04598 beschreibt die Herstellung einer Carotinoidzusammensetzung, enthaltend ein Carotinoid in einem Öl, eine Dispersion eines wasserdispergierbaren Matrixbildners, beispielsweise ein Zucker, und eines Stabilisators, beispielsweise Gelatine oder Casein und einen Emulgator sowie gegebenenfalls ein nicht-öliges Lösungsmittel, wie Glycerin.

Die WO 97/26802 beschreibt gebrauchsfertige wasserdispergierbare Pigmentzusammensetzungen, in denen ein hydrophobes Pigment natürlichen Ursprungs ohne Zusatz von Tensiden in einer wässrigen Phase, die ein Hydrokolloid enthält, dispergiert vorliegt.

In EP 0 795 585 werden wässrige feinteilige Carotinoidsuspensionen beschrieben, die kein Schutzkolloid sondern mindestens einen speziellen Emulgator enthalten. Der Farbton der erhalten Suspensionen variiert aufgrund der unterschiedlichen Teilchengröße.

In EP 0 832 569 wird die Herstellung Carotinoid-haltiger, kaltwasserdispergierbarer Trockenpulver beschrieben, wobei eine Dispersion eines feinteiligen Carotinoids thermisch behandelt wird, um einen gewünschten Farbton einzustellen.

Die WO 2008/087140 beschreibt flüssige Formulierungen, enthaltend wenigstens ein Carotinoid, wenigstens ein hydrophiles Schutzkolloid und wenigstens einen mit Wasser mischbaren Alkohol.

Kommerziell erhältliche pulverförmige Formulierungen von Carotinoiden weisen eine gute Lagerstabilität auf und zeigen gute Färbeeigenschaften in den verschiedenen Getränkeanwendungen, wie beispielsweise Fruchtsäften, Limonaden, Sportlergetränken, Milchgetränken oder Vitamingetränken. Für die Anwendung in mineralienreichen Sportlergetränken oder bei Verwendung von Trinkwasser mit einem hohen Gehalt an Calcium- oder Magnesiumionen dürfen die in Flaschen abgefüllten Getränke keine Ringbildung (Aufrahmung), sowie keine Abscheidung der Carotinoidkomponente an der Flaschenwand zeigen.

Nachteilig bei der Verwendung von pulverförmigen Formulierungen von Carotinoiden sind die Gefahr einer Staubentwicklung und damit verbunden eine Verunreinigung der Umgebung, die Gefahr der elektrostatischen Aufladung der Pulver bei Umfüllprozessen sowie die Gefahr der Schaum- oder Klumpenbildung beim Auflösen der Pulver in Wasser. Ebenfalls ist es nachteilig, dass die pulverförmigen Formulierungen von Carotinoiden üblicherweise als wässrige Stammlösungen mit einem Carotinoidgehalt von ca. 0,1 Gew.-% eingesetzt werden, wodurch immer auch merkliche Wassermengen dem zu färbendem Lebensmittel zugeführt werden.

Der vorliegenden Erfindung lag die Aufgabe zugrunde, eine gebrauchsfertige, stabile Formulierung von β-Carotin zur Verfügung zu stellen, die die wünschenswerten Eigenschaften der kommerziell erhältlichen pulverförmigen Formulierungen aufweist, wie beispielsweise eine gute Lagerstabilität im Hinblick auf mikrobiologischen Befall, Farbtonkonstanz während der Lagerung, eine gute thermische Stabilität und Unempfindlichkeit im Einsatz in Calcium- oder Magnesium-haltigen Getränken oder bei Verwendung von Trinkwasser mit einem hohen Gehalt an Calcium- oder Magnesiumionen, jedoch nicht die verarbeitungstechnischen Nachteile einer pulverförmigen Formulierung zeigt. Eine weitere Aufgabe der vorliegenden Erfindung lag in der Bereitstellung einer Formulierung von β-Carotin mit den oben genannten Eigenschaften, wobei die Formulierung zur Erzeugung eines orange gefärbten und mit Provitamin A angereicherten Lebensmittels, insbesondere eines Getränkes eingesetzt werden kann, und wobei der Farbton von gelborange über orange bis rotorange reichen sollte.

Diese Aufgabe wird durch eine gebrauchsfertige, stabile Suspension teilamorpher β-Carotinpartikel, enthaltend:

| | |
|---|---|
| 1 bis 20 Gew.-% | β-Carotin, |
| 0,2 bis 20 Gew.-% | eines essbaren Öles, |
| 3 bis 60 Gew.-% | Octenyl-Succinat Stärke, |
| 5 bis 60 Gew.-% | eines physiologisch verträglichen Polyalkohols und |
| 5 bis 60 Gew.-% | Wasser, |

wobei sich die Gew.-%-Angaben auf die gebrauchsfertige, stabile Suspension beziehen, die Summe der Anteile des β-Carotins, des essbaren Öles, der Octenyl-Succinat Stärke, des physiologisch verträglichen Polyalkohols und des Wassers zusammen mindestens 80 Gew.-% beträgt, und das Gewichtsverhältnis zwischen β-Carotin und essbarem Öl von 1 : 0,01 bis 1 : 2 reicht,

### gelöst.

Unter dem Merkmal "gebrauchsfertig" wird im Rahmen der vorliegenden Erfindung die Eigenschaft verstanden, dass die erfindungsgemäße Suspension direkt für ihre Zwecke vom Verwender eingesetzt werden kann, sie also direkt dosierbar ist, beispielsweise in der Lebensmittelindustrie zum Anfärben von Lebensmitteln oder zur Anreicherung von Lebensmitteln mit Provitamin A (β-Carotin), wobei sich ein Farbton von gelborange bis rotorange erzeugen lässt.

Unter dem Merkmal "stabil" wird im Rahmen der vorliegenden Erfindung die die mikrobielle, kolloidale und chemische Stabilität verstanden. Bei der mikrobiellen Stabilität handelt sich um einen bakteriostatischen Effekt. Die kolloidale Stabilität bezieht sich auf eine nicht einsetzende Phasentrennung und Änderung der Farbcharakteristik. Die chemische Stabilität bezieht sich auf eine stabile Farbcharakteristik und den β-Carotingehalt.

Die erfindungsgemäße gebrauchsfertige, stabile Suspension enthält 1 bis 20 Gew.-%, bevorzugt 3 bis 15 Gew.-%, besonders bevorzugt 5 bis 10 Gew.-% β-Carotin.

Bevorzugt ist das in der erfindungsgemäßen gebrauchsfertigen, stabilen Suspension enthaltende β-Carotin synthetisch hergestellt.

Die erfindungsgemäße gebrauchsfertige, stabile Suspension enthält 0,2 bis 20 Gew.-%, bevorzugt 0,5 bis 10 Gew.-%, besonders bevorzugt 1 bis 5 Gew.-% eines essbaren Öles.

Als essbare Öle kommen prinzipiell physiologisch verträgliche Öle synthetischer, mineralischer, pflanzlicher oder tierischer Herkunft in Frage. Beispiele sind Sesamöl, Maiskeimöl, Baumwollsaatöl, Sojabohnenöl, Erdnussöl, Ester mittel-kettiger pflanzlicher Fettsäuren, Oleostearin, Paraffinöl, Glycerylstearat, Isopropylmyristat, Diisopropyladipat, 2-Ethylhexansäurecetylstearylester, hydriertes Polyisobuten, Caprylsäure/Caprinsäure-Triglyceride, Palmöl, Palmkernöl, Lanolin und PUFAs (polyunsaturated fatty acids, wie Eicosapentaensäure (EPA), Docosahexaensäure (DHA) und alpha-Linolensäure.

Bevorzugt ist das essbare Öl in der erfindungsgemäßen gebrauchsfertigen, stabilen Suspension pflanzlicher oder tierischer Herkunft, wobei es bei 30 °C flüssig ist, wie Sonnenblumenöl, Palmöl, Palmkernöl, Sesamöl, Maiskeimöl, Baumwollsaatöl, Sojabohnenöl, Erdnussöl, Ester mittelkettiger Triglyceride (sogenannte MCT Öle), Fischöle, wie Makrelen-, Sprotten- oder Lachsöl.

Besonders bevorzugt ist das essbare Öl in der erfindungsgemäßen gebrauchsfertigen, stabilen Suspension ein Pflanzenöl. Ganz besonders bevorzugt ist das essbare Öl ein mittelkettiges Triglycerid (MCT-Öl). Mittelkettige Triglyceride enthalten weitestgehend nur gesättigte Fettsäuren wie sie beispielsweise in Palmkernöl oder Kokosöl enthalten sind.

In der erfindungsgemäßen gebrauchsfertigen, stabilen Suspension reicht das Gewichtsverhältnis zwischen β-Carotin und essbarem Öl von 1 : 0,01 bis 1 : 2, bevorzugt von 1 : 0,05 bis 1 : 1,5, besonders bevorzugt von 1 : 0,2 bis 1 : 1, insbesondere von 1 : 0,3 bis 1 : 0,7. Bei den beschriebenen Gewichtsverhältnissen von β-Carotin zu essbarem Öl liegen üblicherweise mehr als 50 % des eingesetzten β-Carotins als Feststoff vor und ein geringerer Anteil, das heißt weniger als 50 %, des eingesetzten β-Carotins ist im vorhandenen essbaren Öl gelöst.

Das β-Carotin und das essbare Öl bilden den Hauptbestandteil der hydrophoben Phase der erfindungsgemäßen Suspension, wobei nach jetzigem Kenntnisstand ein Teil des Öls mit den festen β-Carotinpartikeln zusammenhängt, und ein Teil des Öls in Tröpfchenform vorliegt, wobei diese Öltröpfchen nur molekular gelöstes β-Carotin und keine β-Carotinpartikel enthalten.

Die festen β-Carotinpartikel sind nicht vollständig kristallin, sondern sie sind teilamorph. In der vorliegenden Erfindung wird unter teilamorph ein Kristallinitätsgrad von weniger als 95% verstanden, bevorzugt ein Kristallinitätsgrad von weniger als 70%. Der kristalline bzw. der amorphe Anteil in den β-Carotinteilchen kann beispielsweise durch Röntgenbeugungsmessungen bestimmt werden. Der röntgenamorphe Anteil in den β-Carotinteilchen der erfindungsgemäßen Suspension liegt bevorzugt zwischen 50 und 100 %, insbesondere zwischen 60 und 100 %.

Die erfindungsgemäße gebrauchsfertige, stabile Suspension enthält 3 bis 60 Gew.-%, bevorzugt 5 bis 40 Gew.-%, insbesondere 7 bis 30 Gew.-% Octenyl-Succinat Stärke.

Octenyl-Succinat Stärke ist im Handel unter der Bezeichnung Purity Gum 2000 von National Starch oder Clear Gum CO 01 von Roquette, Hi Cap 100 oder Capsul von National Starch erhältlich.

Bevorzugter Vertreter aus dieser Gruppe der Octenyl-Succinat Stärken ist Purity^{®} Gum 2000.

Die erfindungsgemäße gebrauchsfertige, stabile Suspension enthält 5 bis 60 Gew.-%, bevorzugt 15 bis 50 Gew.-%, besonders bevorzugt 25 bis 45 Gew.-%, insbesondere 30 bis 40 Gew.-% eines physiologisch verträglichen Polyalkohols.

Der in der erfindungsgemäßen Suspension vorhandene physiologisch verträgliche Polyalkohol weist üblicherweise einen Siedepunkt von mehr als 150 °C, bevorzugt von mehr als 180 °C, insbesondere von mehr als 200 °C bei Normaldruck auf.

Bei dem physiologisch verträglichen Polyalkohol handelt es sich bevorzugt um Glycerin, Monoester des Glycerins mit C₁-C₅-Monocarbonsäuren, Monoether des Glycerin, Propylenglycol oder Sorbitol. Besonders bevorzugt wird Glycerin als physiologisch verträglicher Polyalkohol.

Die erfindungsgemäße gebrauchsfertige, stabile Suspension enthält 5 bis 60 Gew.-%, bevorzugt 15 bis 50 Gew.-%, besonders bevorzugt 25 bis 45 Gew.-%, insbesondere 30 bis 40 Gew.-% Wasser.

In der erfindungsgemäßen gebrauchsfertigen, stabilen Suspension beträgt die Summe der Anteile des β-Carotins, des essbaren Öles, der Octenyl-Succinat Stärke, des physiologisch verträglichen Polyalkohols und des Wassers zusammen mindestens 80 Gew.-%, bevorzugt mindestens 90 Gew.-%, insbesondere mindestens 95 Gew.-%.

In der erfindungsgemäßen, gebrauchsfertigen, stabilen Suspension ist der Gehalt einer weiteren Substanz mit emulgierender Wirkung aus der Klasse der Tenside (niedermolekulare Verbindungen) neben der Octenyl-Succinat Stärke üblicherweise kleiner als 2 Gew.-%, bevorzugt kleiner als
1 Gew.-%. Tenside als Substanzen mit emulgierender Wirkung sind beispielsweise Ascorbylpalmitat, Polyglycerin-Fettsäureester, wie Polyglycerin-3-polyricinoleat (PGPR 90), Sorbitan-Fettsäureester, wie Sorbitanmonostearat (Span 60), PEG(20)-Sorbitolmonooleat, Propylenglykol-Fettsäureester, Saccharoseester von langkettigen Fettsäuren, wie beispielsweise Saccharosemonopalmitat, oder Phospholipide, wie Lecithin.

Handelt es sich bei der weiteren Substanz mit emulgierender Wirkung um Ascorbylpalmitat, so ist der Gehalt an Ascorbylpalmitat bevorzugt geringer als 0,5 Gew.-%, besonders bevorzugt geringer als 0,25 Gew.-%, insbesondere geringer als 0,1 Gew.-%, wobei die Gew.-%-Angaben sich auf das Gesamtgewicht der Suspension beziehen.

Zur Erhöhung der Stabilität des β-Carotins in der erfindungsgemäßen, gebrauchsfertigen, stabilen Suspension gegen oxidativen Abbau ist es vorteilhaft, Stabilisatoren wie d,l-alpha-Tocopherol, t-Butylhydroxytoluol, t-Butylhydroxyanisol, Ascorbinsäure, Salze der Ascorbinsäure oder Ethoxyquin als Antioxidationsmittel zuzusetzen.

Bevorzugt enthält die gebrauchsfertige, stabile Suspension teilamorpher β-Carotinpartikel zusätzlich 0,1 bis 5 Gew.-%, bevorzugt 0,2 bis 4 Gew.-% mindestens eines Antioxidationsmittels, wobei sich die Gew.-%-Angaben auf die gebrauchsfertige, stabile Suspension beziehen. Besonders bevorzugt wird der erfindungsgemäßen, gebrauchsfertigen, stabilen Suspension 0,5 bis 2,0 Gew.-% d,l-alpha-Tocopherol als Antioxidationsmittels zugesetzt.

Besonders bevorzugt ist eine erfindungsgemäße, gebrauchsfertige, stabile Suspension enthaltend,

| | |
|---|---|
| 3 bis 10 Gew.-% | β-Carotin, |
| 1 bis 5 Gew.-% | mittelkettiges Triglycerid als essbares Öl, |
| 0,5 bis 2,0 Gew.-% | d,l-alpha-Toccopherol |
| 10 bis 24 Gew.-% | Octenyl-Succinat Stärke, |
| 30 bis 40 Gew.-% | Glycerin als physiologisch verträglichen Polyalkohol und |
| 30 bis 40 Gew.-% | Wasser, |

wobei die Summe der Anteile von β-Carotin, mittelkettigem Triglycerid, d,l-alpha-Toccopherol, Octenyl-Succinat Stärke, Glycerin und Wasser zusammen mindestens 95 Gew.-% beträgt, und das Gewichtsverhältnis zwischen β-Carotin und mittelkettigem Triglycerid von 1 : 0,3 bis 1 : 0,7 reicht.

In der erfindungsgemäßen, gebrauchsfertigen, stabilen Suspension teilamorpher β-Carotinpartikel liegen die teilamorphen β-Carotinpartikel bevorzugt als nanopartikuläre Teilchen vor.

Als nanopartikuläre Teilchen sind solche Teilchen zu verstehen, die eine über Fraunhofer Beugung ermittelte mittlere Teilchengröße D[4,3] von 0,02 bis 100 µm, bevorzugt 0,05 bis 50 µm, besonders bevorzugt 0,05 bis 20 µm, ganz besonders bevorzugt 0,05 bis 5 µm, insbesondere 0,05 bis 1,0 µm aufweisen. Der Begriff D[4,3] bezeichnet den volumengewichteten mittleren Durchmesser (siehe Handbuch zu Malvern Mastersizer S, Malvern Instruments Ltd., UK), welcher mittels Fraunhofer Beugung bestimmt werden kann.

Bevorzugt weisen die β-Carotinteilchen in der erfindungsgemäßen Suspension eine mittlere Teilchengröße D[4,3] von 0,05 bis 0,8 µm, bevorzugt 0,1 bis 0,7 µm, insbesondere 0,4 bis 0,6 µm auf.

Besonders bevorzugt weisen die β-Carotinpartikel in der erfindungsgemäßen gebrauchsfertigen, stabilen Suspension eine mittlere Teilchengröße von 0,05 bis 0,8 µm, bevorzugt 0,1 bis 0,7 µm, insbesondere 0,4 bis 0,6 µm und durchschnittlich einen röntgenamorphen Anteil zwischen 50 und 100 %, bevorzugt zwischen 60 und 100 % auf.

Ein weiterer Gegenstand der Erfindung ist auch ein Verfahren zur Herstellung einer gebrauchsfertigen, stabilen Suspension teilamorpher β-Carotinpartikel enthaltend:

| | |
|---|---|
| 1 bis 20 Gew.-% | β-Carotin, |
| 0,2 bis 20 Gew.-% | eines essbaren Öles, |
| 3 bis 60 Gew.-% | Octenyl-Succinat Stärke, |
| 5 bis 60 Gew.-% | eines physiologisch verträglichen Polyalkohols und |
| 5 bis 60 Gew.-% | Wasser, |

wobei sich die Gew.-%-Angaben auf die gebrauchsfertige, stabile Suspension beziehen, die Summe der Anteile des β-Carotins, des essbaren Öles, der Octenyl-Succinat Stärke, des physiologisch verträglichen Polyalkohols und des Wassers zusammen mindestens 80 Gew.-% beträgt, und das Gewichtsverhältnis zwischen β-Carotin und essbarem Öl von 1 : 0,01 bis 1 : 2 reicht,
umfassend die Schritte
a₁) Lösen des β-Carotins zusammen mit dem essbaren Öl in einem mit Wasser mischbaren, organischen Lösungsmittel oder in einer Mischung aus Wasser und einem mit Wasser mischbaren, organischen Lösungsmittel bei Temperaturen größer als 30°C, oder
a₂) Lösen des β-Carotinszusammen mit dem essbaren Öl in einem mit Wasser nicht mischbaren, organischen Lösungsmittel,
b) Mischen der nach a₁) oder a₂) erhaltenen Lösung mit einer molekular-dispersen oder kolloiddispersen Lösung der Octenyl-Succinat Stärke in einem Gemisch aus Wasser und dem physiologisch verträglichen Polyalkohol, wobei die β-Carotinhaltige hydrophobe Phase als nanodisperse Phase entsteht,
c) Entfernung der in a₁) oder a₂) verwendeten organischen Lösungsmittel und Aufkonzentration der gebildeten Suspension auf den gewünschten Gehalt an β-Carotin.

Bevorzugte Ausführungsformen hinsichtlich der Komponenten β-Carotin, essbares Öl, Octenyl-Succinat Stärke, physiologisch verträglicher Polyalkohol und Wasser sowie ihren Einsatzmengen finden sich in den bereits eingangs gemachten Erläuterungen.

Die in der Stufe a₁) des erfindungsgemäßen Verfahrens verwendeten wassermischbaren Lösungsmittel sind vor allem wassermischbare, thermisch stabile, flüchtige, nur Kohlenstoff, Wasserstoff und Sauerstoff enthaltenene Lösungsmittel wie Alkohole, Ether, Ester, Ketone oder Acetale. Zweckmäßig verwendet man solche Lösungsmittel, die mindestens zu 10% wassermischbar sind, einen Siedepunkt unter 200°C aufweisen und/oder weniger als 10 Kohlenstoffatome haben. Besonders bevorzugt werden Methanol, Ethanol, n-Propanol, Isopropanol, 1,2-Butandiol-1-methylether (1-Methoxybutanol-2), 1,2-Propandiol-1-n-propylether (1-Propoxy-propanol-2), Tetrahydrofuran oder Aceton verwendet.

Der Begriff "ein mit Wasser nicht mischbares organisches Lösungsmittel" steht im Sinne der vorliegenden Erfindung für ein organisches Lösungsmittel mit einer Wasserlöslichkeit bei Normaldruck von weniger als 10 %. Als mögliche Lösungsmittel kommen dabei u.a. halogenierte aliphatische Kohlenwasserstoffe, wie z. B. Methylenchlorid, Chloroform und Tetrachlorkohlenstoff, Carbonsäureester wie Dimethylcarbonat, Diethylcarbonat, Propylencarbonat, Ethylformiat, Methyl-, Ethyl- oder Isopropylacetat sowie Ether wie Methyl-tert.-butylether in Frage. Bevorzugte, mit Wasser nicht mischbare organische Lösungsmittel sind die folgenden Verbindungen aus der Gruppe, bestehend aus Dimethylcarbonat, Propylencarbonat, Ethylformiat, Ethylacetat, Isopropylacetat und Methyl-tert.-butylether.

In dem erfindungsgemäßen Verfahren wird bevorzugt Verfahrensschritt a₁) ausgeführt, wobei das β-Carotin zusammen mit dem essbaren Öl in einem mit Wasser mischbaren, organischen Lösungsmittel oder in einer Mischung aus Wasser und einem mit Wasser mischbaren, organischen Lösungsmittel bei Temperaturen größer als 30°C, vorzugsweise zwischen 50°C und 240°C, insbesondere 100°C bis 200°C, besonders bevorzugt 140°C bis 180°C, gegebenenfalls unter Druck, gelöst wird.

Da die Einwirkung hoher Temperaturen unter Umständen den gewünschten hohen all-trans Isomerenanteil des β-Carotins herabsetzen kann, löst man das β-Carotin möglichst rasch, beispielsweise im Sekundenbereich, z. B. in 0,1 bis 10 Sekunden, besonders bevorzugt in weniger als 1 Sekunde. Zur raschen Herstellung der molekulardispersen Lösung kann die Anwendung von erhöhtem Druck, z. B. im Bereich von 20 bar bis 80 bar, vorzugsweise 30 bis 60 bar, vorteilhaft sein.

Die so erhaltene molekulardisperse Lösung versetzt man anschließend in Verfahrensschritt b) direkt mit der gegebenenfalls gekühlten molekular-dispersen oder kolloiddispersen Lösung der Octenyl-Succinat Stärke in einem Gemisch aus Wasser und dem physiologisch verträglichen Polyalkohol, wobei die Lösungsmittelkomponente aus Verfahrensschritt a₁) in die wässrige Phase überführt wird und die hydrophobe Phase des β-Carotins zusammen mit dem essbaren Öl als nanodisperse Phase entsteht. Bevorzugt stellt sich in Verfahrensschritt b) eine Mischungstemperatur von etwa 35°C bis 80°C ein.

Hinsichtlich einer näheren Verfahrens- und Apparatebeschreibung zur oben genannten Dispergierung wird an dieser Stelle auf EP-B-0 065 193 Bezug genommen.

In dem erfindungsgemäßen Verfahren werden in Verfahrensschritt c) die in Verfahrensschritt a₁) oder a₂) verwendeten organischen Lösungsmittel entfernt und die gebildete Suspension durch Entfernen von überschüssigem Wasser auf den gewünschten Gehalt an β-Carotin aufkonzentriert. Der verwendete physiologisch verträgliche Polyalkohol wird auf Grund seines hohen Siedepunktes bei diesem Aufkonzentrationsschritt praktisch nicht entfernt.

Die erfindungsgemäße gebrauchsfertige, stabile Suspension teilamorpher β-Carotinpartikel zeichnet sich dadurch aus, dass sie gegenüber den aus pulverförmigen β-Carotinformulierungen herstellbaren wässrigen Stammlösungen im Hinblick auf den β-Carotingehalt wesentlich konzentrierter ist und damit auch Getränkegrundstoffen zugesetzt werden kann, die nur geringe Wassermengen tolerieren. Die erfindungsgemäße Suspension lässt sich problemlos dosieren und zeigt keine unerwünschte Sedimentation.

Weiterhin weist die erfindungsgemäße Suspension eine gute Lagerstabilität im Hinblick auf mikrobiologischen Befall und im Hinblick auf die Farbtonkonstanz während der Lagerung auf. Die erfindungsgemäße Suspension zeigt außerdem eine gute thermische Stabilität und die geforderte Unempfindlichkeit im Einsatz in Calcium- oder Magnesium-haltigen Getränken oder bei Verwendung von Trinkwasser mit einem hohen Gehalt an Calcium- oder Magnesiumionen. Die mit der erfindungsgemäßen Suspension versetzten Getränke weisen eine gute Stabilität hinsichtlich einer unerwünschten Ringbildung (Aufrahmung) auf.

Die erfindungsgemäße gebrauchsfertige, stabile Suspension teilamorpher β-Carotinpartikel eignet sich u.a. als Zusatzstoff zu Lebensmittelzubereitungen, beispielsweise zur Färbung von Lebensmitteln wie Getränken, als Mittel für die Herstellung pharmazeutischer und kosmetischer Zubereitungen sowie für die Herstellung von Nahrungsergänzungspräparaten, beispielsweise von Multivitaminpräparaten im Human- und Tierbereich. Bevorzugt eignet sich die erfindungsgemäße gebrauchsfertige, stabile Suspension als Zusatz zu Getränken.

Die erfindungsgemäße Suspension weist für β-Carotin einen orangen Farbton auf und eignet sich zum Färben von Lebensmitteln in dem Farbbereich gelborange bis rotorange. Die β-Carotin-haltige Suspension dient außerdem zur Anreicherung von Lebensmitteln mit Provitamin A.

Ein weiter Gegenstand der vorliegenden Erfindung ist daher auch die Verwendung der oben beschriebenen, erfindungsgemäßen gebrauchsfertigen, stabilen Suspension teilamorpher β-Carotinpartikel als Zusatz zu Tierfuttermitteln, Lebensmitteln, Nahrungsergänzungsmitteln sowie kosmetischen und pharmazeutischen Mitteln, insbesondere auch als Zusatz bei der Herstellung von Getränken, wobei bevorzugt die erfindungsgemäße Suspension direkt dem Getränk zugesetzt wird.

Ebenfalls Gegenstand der vorliegenden Erfindung sind Tierfuttermittel, Lebensmittel und Nahrungsergänzungsmittel, insbesondere ein Getränk, welche die erfindungsgemäße, gebrauchsfertige, stabile Suspension teilamorpher β-Carotinpartikel enthält.

Ein weiterer Gegenstand der vorliegenden Erfindung ist die Verwendung der oben beschriebenen gebrauchsfertigen, stabilen Suspension von β-Carotin, zum Anreichern von Tierfuttermitteln, Lebensmitteln und Nahrungsergänzungsmitteln sowie kosmetischen und pharmazeutischen Mitteln mit Provitamin A

Ebenfalls Gegenstand der vorliegenden Erfindung ist ein Verfahren zur Anreicherung von Tierfuttermitteln, Lebensmitteln und Nahrungsergänzungsmitteln sowie kosmetischen und pharmazeutischen Mitteln, insbesondere von Getränken, mit Provitamin A durch Zugabe der erfindungsgemäßen, gebrauchsfertigen, stabilen Suspension teilamorpher β-Carotinpartikel zu denselben.

Die Erfindung wird durch folgende, die Erfindung jedoch nicht einschränkende Beispiele erläutert:

### Beispiele

### Beispiel 1: Herstellung einer β-Carotin-Suspension

In einer beheizbaren Vorlage wurden bei einer Temperatur von 30 °C 57 g kristallines β-Carotin, 27 g MCT-Öl und 8,4 g α-Tocopherol in 315 g eines azeotropen Isopropanol/Wasser-Gemischs suspendiert. Die Wirkstoffsuspension wurde dann auf 73 °C erwärmt und bei einer Flussrate von 3,4 kg/h kontinuierlich mit weiterem Isopropanol/Wasser-Azeotrop der Temperatur 235 °C und einer Flussrate von 4,8 kg/h in einer Mischkammer vermischt, wobei sich das β-Carotin bei einer sich einstellenden Mischungstemperatur von 171 °C bei einem Druck von 65 bar löste. Diese Wirkstofflösung wurde unmittelbar in einer zweiten Mischkammer mit einer wässrigen Lösung von 227 g Purity Gum 2000, 286 g Glycerin in 6646 g destilliertem Wasser bei einer Flussrate von 60 kg/h vermischt.
Die bei der Mischung entstandenen Wirkstoffteilchen wiesen im Isopropanol/WasserGemisch eine Teilchengröße von 438 nm auf, bei einem E1/1-Wert von 115 (E1/1: Extinktion einer 1 gew.-%igen Suspension in einer 1 cm Küvette).
Anschliessend wurde die Wirkstoff-Suspension am Dünnfilmverdampfer auf eine Konzentration von ca. 6,2 Gew.-% Wirkstoffgehalt aufkonzentriert.

Die in Beispiel 1 hergestellte Suspension, die einen β-Carotin-Gehalt von
ca. 6,2 % Gew.-% aufwies, wurde in den nachfolgenden, anwendungstechnischen Untersuchungen mit der Bezeichnung OL gekennzeichnet.

### Beispiel 2: Sportgetränk ohne Fruchtsaft (15 ppm β-Carotin)

| Zutaten | eingesetzte Menge in g/L |
|---|---|
| Ascorbinsäure | 0,150 |
| Calciumhydrogenphosphat | 0,215 |
| Carboxymethylcellulose | 2,000 |
| Kaliumdihydrogenphosphat | 0,350 |
| Tri-Natriumcitrat | 0,486 |
| Natriumbenzoat | 0,150 |
| Kaliumsorbat | 0,200 |
| Zitronensäure (wasserfrei) | 2,500 |
| Saccharose | 61,000 |
| H₂O | ad 1000 |
| OL berechnet als β-Carotin | 0,015 |

Nach dem Ausmischen der Inhaltstoffe wurde das Getränk bei 90 °C für 60 s pasteurisiert.

### Vergleichsbeispiel 2b

Analog wie in Beispiel 2 beschrieben wurde ein Sportgetränk zubereitet, wobei anstelle von der erfindungsgemäßen Suspension OL aus Beispiel 1 eine Stammlösung mit einem β-Carotin-Gehalt von 0,1 Gew.-%, zubereitet aus dem pulverförmigen kommerziell erhältlichen Produkt Lucarotin^{®} 10 CWD/O (10 Gew.-% β-Carotin), als β-Carotin-Quelle eingesetzt wurde.

Ergebnis:
Es wurde festgestellt, dass die chemische Stabilität (Beta-Carotin Gehalt, Farbe) des in Beispiel 2 hergestellten Sportgetränkes keinen Nachteil gegenüber dem in Vergleichsbeispiel 2b hergestellten Sportgetränkes aufweist.

### Beispiel 3: Milchgetränk (5 ppm β-Carotin)

| Zutaten | eingesetzte Menge in g/L |
|---|---|
| H₂O | ad 1000 |
| Orangesaftkonzentrat (54 °Brix) | 15,046 |
| Fettarme Milch 0,1 % Fett | 3,636 |
| Stabilisator Pektin | 3,314 |
| Zitronensäure (wasserfrei) | 6,179 |
| Äpfelsäure | 1,612 |
| Calciumchlorid | 1,075 |
| Triatriumcitrat | 1,881 |
| Saccharose | 107,464 |
| Kaliumsorbat | 0,445 |
| Natriumbenzoat | 0,493 |
| OL berechnet als β-Carotin | 0,005 |

Nach der Ausmischung wurde das Getränk bei 200 bar hochdruckhomogenisiert und bei 90 °C für 60 s pasteurisiert.

### Beispiel 4: Alkopop (3,5 ppm β-Carotin)

| Zutaten | eingesetzte Menge in g/L |
|---|---|
| Neutral Sprit (23,8 % Vol.) | 188,810 |
| Saccharose | 49,260 |
| Orangensaftkonzentrat (54 °Brix) | 98,820 |
| Zitronensäure | 1,000 |
| OL berechnet als β-Carotin | 0,0035 |
| Ascorbinsäure | 0,150 |
| Natriumbenzoat | 0,500 |
| Kaliumsorbat | 0,500 |
| H₂O | ad 1000 |

Nach der Ausmischung wurde das Getränk bei 200 bar hochdruckhomogenisiert und bei 65 °C für 60 s pasteurisiert.

### Vergleichsbeispiel 4b

Analog wie in Beispiel 4 beschrieben wurde ein Alkopop zubereitet, wobei anstelle von der erfindungsgemäßen Suspension OL aus Beispiel 1 eine Stammlösung mit einem β-Carotin-Gehalt von 0,1 Gew.-%, zubereitet aus dem pulverförmigen kommerziell erhältlichen Produkt Lucarotin^{®} 10 CWD/O, als β-Carotin-Quelle eingesetzt wurde.

Ergebnis:
Es wurde festgestellt, dass die chemische Stabilität (Beta-Carotin Gehalt, Farbe) des in Beispiel 4 hergestellten Alkopops keinen Nachteil gegenüber dem in Vergleichsbeispiel 4b hergestellten Alkopop aufweist.

### Beispiel 5: Vitamingetränk mit 30 % Fruchtsaft (15 ppm β-Carotin)

| Zutaten | eingesetzte Menge in g/L |
|---|---|
| Saccharose | 85,300 |
| Pektin | 0,200 |
| Natriumbenzoat | 0,200 |
| Kaliumsorbat | 0,200 |
| Ascorbinsäure | 0,300 |
| Zitronensäure 50 Gew.-% | 5,000 |
| Calcium-Lactat-5-Hydrat | 4,500 |
| H'2O | ad 1000 |
| Orangensaftkonzentrat (54 °Brix) | 64,230 |
| OL berechnet als β-Carotin | 0,015 |
| Vitamin E 500 BG | 0,070 |

Nach der Ausmischung wurde das Getränk bei 200 bar hochdruckhomogenisiert und bei 90 °C für 60 s pasteurisiert.

### Beispiel 6: Vitamingetränk mit 100 % Fruchtsaft (30 ppm β-Carotin)

| Zutaten | eingesetzte Menge in g/L |
|---|---|
| Orangensaftkonzentrat (54 °Brix) | 220,000 |
| H₂O | ad 1000 |
| Natriumbenzoat | 1,000 |
| Ascorbinsäure | 0,400 |
| OL berechnet als β-Carotin | 0,03 |

Nach der Ausmischung wurde das Getränk bei 100 bar hochdruckhomogenisiert und bei 90 °C für 60 s pasteurisiert.

### Beispiel 7: Orangensaftgrundstoff im Cold-Blend-Verfahren (30 ppm β-Carotin)

| Zutaten | eingesetzte Menge in g/L |
|---|---|
| Orangensaftkonzentrat (54 °Brix) | 840,000 |
| Natriumbenzoat | 3,500 |
| H₂O | ad 1000 |
| Ascorbinsäure | 1,500 |
| OL berechnet als β-Carotin | 0,030 |

Im Cold-Blend-Verfahren wurden alle Zutaten zur Herstellung des Orangensaftgrundstoffes in -2 °C kaltes Orangensaftkonzentrat eingearbeitet. Die hohe Viskosität des Getränkemediums sowie die niedrige Temperatur stellte besondere Ansprüche an die β-Carotin-Formulierung hinsichtlich Stabilität und Verarbeitbarkeit.

### Beispiel 8: Salatdressing (6,2 ppm β-Carotin)

| Zutaten | eingesetzte Menge in g/L |
|---|---|
| Xanthan | 30,000 |
| Senf-Pulver | 12,500 |
| Natriumchlorid | 40,000 |
| Essig | 90,000 |
| Saccharose | ad 1000 |
| H₂O | 352,494 |
| Pflanzliches Öl | 360,000 |
| OL berechnet als β-Carotin | 0,0062 |

Die Zutaten wurden gemischt, mit einem Ultra-Turrax homogenisiert und erhitzt.

### Beispiel 9: Pudding (2 ppm β-Carotin)

| Zutaten | eingesetzte Menge in g/L |
|---|---|
| Maisstärke | 64,000 |
| Saccharose | 72,000 |
| Milch 3,5 % Fett | ad 1000 |
| OL berechnet als β-Carotin | 0,002 |

### Beispiel 10: Eiszubereitung (3 ppm β-Carotin)

| Zutaten | eingesetzte Menge in g/L |
|---|---|
| Milch 3,5 % Fett | ad 1000 |
| Sahne 34 % Fett | 43,103 |
| Saccharose | 196,55 |
| Emulgator Lecithin | 2,586 |
| Magermilchpulver | 41,379 |
| Natriumchlorid | 0,862 |
| OL berechnet als β-Carotin | 0,003 |

### Beispiel 11: Joghurt (5 ppm β-Carotin)

| Zutaten | eingesetzte Menge in g/L |
|---|---|
| Milch 3,5 % Fett | ad 1000 |
| Joghurt | 150,000 |
| OL berechnet als β-Carotin | 0,005 |

## Patentansprüche

1. Gebrauchsfertige, stabile Suspension teilamorpher β-Carotin Partikel, enthaltend:
| | |
|---|---|
| 1 bis 20 Gew.-% | β-Garotin, |
| 0,2 bis 20 Gew.-% | eines essbaren Öles, |
| 3 bis 60 Gew.-% | Octenyl-Succinat Stärke als hydrophiles Schutzkolloid, |
| 5 bis 60 Gew.-% | eines physiologisch verträglichen Polyalkohols und |
| 5 bis 60 Gew.-% | Wasser, |
wobei sich die Gew.-%-Angaben auf die gebrauchsfertige, stabile Suspension beziehen, die Summe der Anteile des β-Carotins, des essbaren Öles, der Octenyl-Succinat Stärke, des physiologisch verträglichen Polyalkohols und des Wassers zusammen mindestens 80 Gew.-% beträgt, und das Gewichtsverhältnis zwischen β-Carotin und essbarem Öl von 1 : 0,01 bis 1 : 2 reicht.

2. Gebrauchsfertige, stabile Suspension teilamorpher β-Carotin Partikel nach Anspruch 1, wobei das essbare Öl ein Pflanzenöl ist.

3. Gebrauchsfertige, stabile Suspension teilamorpher β-Carotin Partikel nach Anspruch 1 oder 2, wobei der physiologisch verträglichen Polyalkohol Glycerin ist.

4. Gebrauchsfertige, stabile Suspension teilamorpher β-Carotin Partikel nach einem der Ansprüche 1 bis 3, welche zusätzlich 0,1 bis 5 Gew.-% mindestens eines Antioxidationsmittels enthält.

5. Gebrauchsfertige, stabile Suspension teilamorpher β-Carotin Partikel nach Anspruch 1 enthaltend,
| | |
|---|---|
| 3 bis 10 Gew.-% | β-Carotin, |
| 1 bis 5 Gew.-% | mittelkettiges Triglycerid als essbares Öl, |
| 0,5 bis 2,0 Gew.-% | d,l-alpha-Toccopheroi |
| 10 bis 24 Gew.-% | Octenyl-Succinat Stärke als hydrophiles Schutzkolloid, |
| 30 bis 40 Gew.-% | Glycerin als physiologisch verträglichen Polyalkohol und |
| 30 bis 40 Gew.-% | Wasser, |
wobei die Summe der Anteile von β-Carotin, mittelkettigem Triglycerid, d,l-alpha-Toccopherol, Octenyl-Succinat Stärke, Glycerin und Wasser zusammen mindestens 95 Gew.-% beträgt, und das Gewichtsverhältnis zwischen β-Carotin und mittelkettigem Triglycerid von 1 : 0,3 bis 1 : 0,7 reicht.

6. Gebrauchsfertige, stabile Suspension teilamorpher β-Carotin Partikel nach einem der Ansprüche 1 bis 5, wobei die β-Carotin Artikel eine mittlere Teilchengröße von 0,05 bis 0,8 µm und durchschnittlich einen röntgenamorphen Anteil zwischen 50 und 100 % aufweisen.

7. Verfahren zur Herstellung einer gebrauchsfertigen, stabilen Suspension teilamorpher β-Carotin Partikel enthaltend:
| | |
|---|---|
| 1 bis 20 Gew.-% | β-Carotin, |
| 0,2 bis 20 Gew.-% | eines essbaren Öles, |
| 3 bis 60 Gew.-% | Octenyl-Succinat Stärke, |
| 5 bis 60 Gew.-% | eines physiologisch verträglichen Polyalkohols und |
| 5 bis 60 Gew.-% | Wasser, |
wobei sich die Gew.-%-Angaben auf die gebrauchsfertige, stabile Suspension beziehen, die Summe der Anteile des β-Carotins, des essbaren Öles, der Octenyl-Succinat Stärke, des physiologisch verträglichen Polyalkohols und des Wassers zusammen mindestens 80 Gew.-% beträgt, und das Gewichtsverhältnis zwischen Carotinoid und essbarem Öl von 1 : 0,01 bis 1 : 2 reicht,
umfassend die Schritte
a₁) Lösen des β-Carotins zusammen mit dem essbaren Öl in einem mit Wasser mischbaren, organischen Lösungsmittel oder in einer Mischung aus Wasser und einem mit Wasser mischbaren, organischen Lösungsmittel bei Temperaturen größer als 30°C, oder
a₂) Lösen des β-Carotins zusammen mit dem essbaren Öl in einem mit Wasser nicht mischbaren, organischen Lösungsmittel,
b) Mischen der nach a₁) oder a₂) erhaltenen Lösung mit einer molekulardispersen oder kolloiddispersen Lösung der Octenyl-Succinat Stärke in einem Gemisch aus Wasser und dem physiologisch verträglichen Polyalkohol, wobei die β-Carotin-haltige hydrophobe Phase als nanodisperse Phase entsteht,
c) Entfernung der in a₁) oder a₂) verwendeten organischen Lösungsmittel und Aufkonzentration der gebildeten Suspension auf den gewünschten Gehalt an β-Carotin.

8. Verwendung der gebrauchsfertigen, stabilen Suspension teilamorpher β-Carotin Partikel nach einem der Ansprüche 1 bis 6 als Zusatz zu Tierfuttermitteln, Lebensmitteln und Nahrungsergänzungsmitteln sowie kosmetischen und pharmazeutischen Mitteln.

9. Verwendung der gebrauchsfertigen, stabilen Suspension teilamorpher β-Carotin Partikel nach einem der Ansprüche 1 bis 6 als Zusatz bei der Herstellung von Getränken.

10. Verwendung nach Anspruch 9, wobei die gebrauchsfertige, stabile Suspension teilamorpher β-Carotin Partikel nach einem der Ansprüche 1 bis 6 direkt dem Getränk zugesetzt wird.

11. Tierfuttermittel, Lebensmittel und Nahrungsergänzungsmittel, umfassend die gebrauchsfertige, stabile Suspension teilamorpher β-Carotin Partikel gemäß einem der Ansprüche 1 bis 6.

12. Getränk, umfassend die gebrauchsfertige, stabile Suspension teilamorpher β-Carotin Partikel gemäß einem der Ansprüche 1 bis 6.

13. Verwendung der gebrauchsfertigen, stabilen Suspension teilamorpher β-Carotin Partikel nach Anspruch 1 zum Anreichern von Tierfuttermitteln, Lebensmitteln und Nahrungsergänzungsmitteln sowie kosmetischen und pharmazeutischen Mitteln mit Provitamin A.

14. Verfahren zur Anreicherung von Tierfuttermitteln, Lebensmitteln und Nahrungsergänzungsmitteln sowie kosmetischen und pharmazeutischen Mitteln, insbesondere von Getränken mit Provitamin A durch Zugabe der gebrauchsfertigen, stabilen Suspension teilamorpher β-Carotin Partikel nach Anspruch 1 zu denselben.

## Claims

1. A ready-to-use, stable suspension of partially amorphous β-carotene particles, comprising:
| | |
|---|---|
| 1 to 20% by weight | of β-carotene, |
| 0.2 to 20% by weight | of an edible oil, |
| 3 to 60% by weight | of octenyl succinate starch as hydrophilic protective colloid, |
| 5 to 60% by weight | of a physiologically tolerated polyhydric alcohol and |
| 5 to 60% by weight | of water, |
wherein the percentages by weight relate to the ready-to-use, stable suspension, the sum of the fractions of the β-carotene, the edible oil, the octenyl succinate starch, the physiologically tolerated polyhydric alcohol and the water together is at least 80% by weight, and the weight ratio between β-carotene and edible oil ranges from 1:0.01 to 1:2.

2. The ready-to-use, stable suspension of partially amorphous β-carotene particles according to claim 1, wherein the edible oil is a vegetable oil.

3. The ready-to-use, stable suspension of partially amorphous β-carotene particles according to claim 1 or 2, wherein the physiologically tolerated polyhydric alcohol is glycerol.

4. The ready-to-use, stable suspension of partially amorphous β-carotene particles according to any one of claims 1 to 3, which additionally comprises 0.1 to 5% by weight of at least one antioxidant.

5. The ready-to-use, stable suspension of partially amorphous β-carotene particles according to claim 1 comprising,
| | |
|---|---|
| 3 to 10% by weight | of β-carotene, |
| 1 to 5% by weight | of medium-chain triglyceride as edible oil, |
| 0.5 to 2.0% by weight | of d,l-alpha-tocopherol |
| 10 to 24% by weight | of octenyl succinate starch as hydrophilic protective colloid, |
| 30 to 40% by weight | of glycerol as physiologically tolerated polyhydric alcohol and |
| 30 to 40% by weight | of water, |
wherein the sum of the fractions of β-carotene, medium-chain triglyceride, d,l-alpha-tocopherol, octenyl succinate starch, glycerol and water together is at least 95% by weight, and the weight ratio between β-carotene and medium-chain triglyceride ranges from 1:0.3 to 1:0.7.

6. The ready-to-use, stable suspension of partially amorphous β-carotene particles according to any one of claims 1 to 5, wherein the β-carotene particles have a median particle size of 0.05 to 0.8 µm and have a mean X-ray amorphous fraction between 50 and 100%.

7. A process for producing a ready-to-use stable suspension of partially amorphous β-carotene particles comprising:
| | |
|---|---|
| 1 to 20% by weight | of β-carotene, |
| 0.2 to 20% by weight | of an edible oil, |
| 3 to 60% by weight | of octenyl succinate starch, |
| 5 to 60% by weight | of a physiologically tolerated polyhydric alcohol and |
| 5 to 60% by weight | of water, |
wherein the percentages by weight relate to the ready-to-use, stable suspension, the sum of the fractions of the β-carotene, the edible oil, the octenyl succinate starch, the physiologically tolerated polyhydric alcohol and the water together is at least 80% by weight, and the weight ratio between carotenoid and edible oil ranges from 1:0.01 to 1:2
comprising the steps
a₁) dissolving the β-carotene together with the edible oil in a water-miscible organic solvent or in a mixture of water and a water-miscible organic solvent at temperatures above 30°C, or
a₂) dissolving the β-carotene together with the edible oil in a water-immiscible organic solvent,
b) mixing the solution obtained according to a₁) or a₂) with a molecularly dispersed or colloidally dispersed solution of the octenyl succinate starch in a mixture of water and the physiologically tolerated polyhydric alcohol, wherein the β-carotene-comprising hydrophobic phase is produced as nanodisperse phase,
c) removing the organic solvent used in a₁) or a₂) and concentrating the suspension formed to the desired content of β-carotene.

8. The use of the ready-to-use, stable suspension of partially amorphous β-carotene particles according to any one of claims 1 to 6 as additive to animal feeds, foods and food supplements and also cosmetic and pharmaceutical compositions.

9. The use of the ready-to-use, stable suspension of partially amorphous β-carotene particles according to any one of claims 1 to 6 as additive in the production of drinks.

10. The use according to claim 9, wherein the ready-to-use, stable suspension of partially amorphous β-carotene particles according to any one of claims 1 to 6 is added directly to the drink.

11. An animal feed, food and food supplement comprising the ready-to-use, stable suspension of partially amorphous β-carotene particles according to any one of claims 1 to 6.

12. A drink comprising the ready-to-use, stable suspension of partially amorphous β-carotene particles according to any one of claims 1 to 6.

13. The use of the ready-to-use, stable suspension of partially amorphous β-carotene particles according to claim 1 for enriching animal feeds, foods and food supplements and also cosmetic and pharmaceutical compositions with provitamin A.

14. A process for enriching animal feeds, foods and food supplements and also cosmetic and pharmaceutical compositions, in particular drinks, with provitamin A by adding the ready-to-use, stable suspension of partially amorphous β-carotene particles according to claim 1 to same.

## Revendications

1. Suspension stable, prête à l'emploi, de particules de β-carotène partiellement amorphes, contenant :
| | |
|---|---|
| 1 à 20 % en poids | de β-carotène, |
| 0,2 à 20 % en poids | d'une huile comestible, |
| 3 à 60 % en poids | d'octényl-succinate d'amidon en tant que colloïde protecteur hydrophile, |
| 5 à 60 % en poids | d'un polyalcool physiologiquement acceptable et |
| 5 à 60 % en poids | d'eau, |
les données en % en poids se rapportant à la suspension stable, prête à l'emploi, la somme des proportions du β-carotène, de l'huile comestible, de l'octényl-succinate d'amidon, du polyalcool physiologiquement acceptable et de l'eau ensemble valant au moins 80 % en poids, et le rapport pondéral entre le β-carotène et l'huile comestible valant de 1 : 0,01 à 1 : 2.

2. Suspension stable, prête à l'emploi, de particules de β-carotène partiellement amorphes selon la revendication 1, dans laquelle l'huile comestible est une huile végétale.

3. Suspension stable, prête à l'emploi, de particules de β-carotène partiellement amorphes selon la revendication 1 ou 2, dans laquelle le polyalcool physiologiquement acceptable est le glycérol.

4. Suspension stable, prête à l'emploi, de particules de β-carotène partiellement amorphes selon l'une quelconque des revendications 1 à 3, qui contient en plus 0,1 à 5 % en poids d'au moins un antioxydant.

5. Suspension stable, prête à l'emploi, de particules de β-carotène partiellement amorphes selon la revendication 1, contenant
| | |
|---|---|
| 3 à 10 % en poids | de β-carotène, |
| 1 à 5 % en poids | de triglycéride à longueur moyenne de chaîne, en tant qu'huile comestible, |
| 0,5 à 2,0 % en poids | de d,l-alpha-tocophérol, |
| 10 à 24 % en poids | d'octényl-succinate d'amidon en tant que colloïde protecteur hydrophile, |
| 30 à 40 % en poids | de glycérol en tant que polyalcool physiologiquement acceptable et |
| 30 à 40 % en poids | d'eau, |
la somme des proportions de β-carotène, triglycéride à longueur moyenne de chaîne, d,l-alpha-tocophérol, octényl-succinate d'amidon, glycérol et eau ensemble valant au moins 95 % en poids, et le rapport pondéral entre le β-carotène et le triglycéride à longueur moyenne de chaîne valant de 1 : 0,3 à 1 : 0,7.

6. Suspension stable, prête à l'emploi, de particules de β-carotène partiellement amorphes selon l'une quelconque des revendications 1 à 5, dans laquelle les particules de β-carotène présentent une taille moyenne de particule de 0,05 à 0,8 µm et en moyenne une proportion amorphe aux rayons X comprise entre 50 et 100 %.

7. Procédé pour la préparation d'une suspension stable, prête à l'emploi, de particules partiellement amorphes de β-carotène contenant :
| | |
|---|---|
| 1 à 20 % en poids | de β-carotène, |
| 0,2 à 20 % en poids | d'une huile comestible, |
| 3 à 60 % en poids | d'octényl-succinate d'amidon, |
| 5 à 60 % en poids | d'un polyalcool physiologiquement acceptable et |
| 5 à 60 % en poids | d'eau, |
les données en % en poids se rapportant à la suspension stable, prête à l'emploi, la somme des proportions du β-carotène, de l'huile comestible, de l'octényl-succinate d'amidon, du polyalcool physiologiquement acceptable et de l'eau ensemble valant au moins 80 % en poids, et le rapport pondéral entre caroténoïde et huile comestible valant de 1 : 0,01 à 1 : 2,
comprenant les étapes
a₁) dissolution du β-carotène, conjointement avec l'huile comestible, dans un solvant organique miscible à l'eau ou dans un mélange constitué d'eau et d'un solvant organique miscible à l'eau, à des températures supérieures à 30 °C, ou
a₂) dissolution du β-carotène, conjointement avec l'huile comestible, dans un solvant organique non miscible à l'eau,
b) mélange de la solution obtenue selon a₁) ou a₂) avec une solution en dispersion moléculaire ou dispersion colloïdale de l'octényl-succinate d'amidon dans un mélange d'eau et du polyalcool physiologiquement acceptable, la phase hydrophobe contenant du β**-**carotène apparaissant sous forme de phase nanodispersée,
c) élimination du solvant organique utilisé en a₁) ou a₂) et concentration de la suspension formée, jusqu'à la teneur désirée en β-carotène.

8. Utilisation de la suspension stable, prête à l'emploi, de particules de β-carotène partiellement amorphes selon l'une quelconque des revendications 1 à 6, en tant qu'additif à des aliments pour animaux, des produits alimentaires et des compléments nutritionnels ainsi qu'à des produits cosmétiques et pharmaceutiques.

9. Utilisation de la suspension stable, prête à l'emploi, de particules de β-carotène partiellement amorphes selon l'une quelconque des revendications 1 à 6, en tant qu'additif dans la fabrication de boissons.

10. Utilisation selon la revendication 9, dans laquelle on ajoute directement à la boisson la suspension stable, prête à l'emploi, de particules de β-carotène partiellement amorphes selon l'une quelconque des revendications 1 à 6.

11. Aliments pour animaux, produits alimentaires et compléments nutritionnels, comprenant la suspension stable, prête à l'emploi, de particules de β-carotène partiellement amorphes selon l'une quelconque des revendications 1 à 6.

12. Boisson, comprenant la suspension stable, prête à l'emploi, de particules de β-carotène partiellement amorphes selon l'une quelconque des revendications 1 à 6.

13. Utilisation de la suspension stable, prête à l'emploi, de particules de β-carotène partiellement amorphes selon la revendication 1, pour l'enrichissement avec de la provitamine A d'aliments pour animaux, de produits alimentaires et de compléments nutritionnels ainsi que de produits cosmétiques et pharmaceutiques.

14. Procédé pour l'enrichissement avec de la provitamine A d'aliments pour animaux, de produits alimentaires et de compléments nutritionnels ainsi que de produits cosmétiques et pharmaceutiques, en particulier de boissons, par addition à ceux-ci de la suspension stable, prête à l'emploi, de particules de β-carotène partiellement amorphes selon la revendication 1.
